(19) Europäisches Patentamt
European Patent Office
Office européen des brevets

(11) Publication number: **0 334 062 B1**

# (12) EUROPEAN PATENT SPECIFICATION

(45) Date of publication of patent specification: **18.05.94**

(51) Int. Cl.$^5$: **A61L 17/00**, A61L 27/00

(21) Application number: **89103601.4**

(22) Date of filing: **01.03.89**

The file contains technical information submitted after the application was filed and not included in this specification

## (54) Bioabsorbable coating for a surgical article.

(30) Priority: **24.03.88 US 172601**
**24.03.88 US 172608**

(43) Date of publication of application:
**27.09.89 Bulletin 89/39**

(45) Publication of the grant of the patent:
**18.05.94 Bulletin 94/20**

(84) Designated Contracting States:
**AT BE CH DE ES FR GB GR IT LI NL SE**

(56) References cited:
**EP-A- 0 214 861**
**EP-A- 0 261 470**
**US-A- 3 942 532**

(73) Proprietor: **AMERICAN CYANAMID COMPANY**
**One Cyanamid Plaza**
**Wayne, NJ 07470-8426(US)**

(72) Inventor: **Jarrett, Peter Kendrick**
**24 Chatfield Drive**
**Trumbull Connecticut 06611(US)**
Inventor: **Casey, Donald James**
**9 Revere Place**
**Ridgefield Connecticut 06877(US)**
Inventor: **Lehmann, Leonard Theodore**
**45 Aunt Hack Road**
**Danbury Connecticut 06811(US)**

(74) Representative: **Wächtershäuser, Günter, Dr.**
**Tal 29**
**D-80331 München (DE)**

EP 0 334 062 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

A bioabsorbable coating for a surgical article comprises a copolymer manufactured from the monomer caprolactone and at least one other copolymerizable monomer. The surgical article can be a bioabsorbable suture or a ligature. The surgical suture or ligature coated with the bioabsorbable copolymer has improved knot repositioning properties.

The bioabsorbable coating of this invention has advantages over prior art coatings used with surgical sutures or ligatures. Specifically, sutures coated with the copolymer coating of this invention are less stiff than sutures using the coating described in the prior art. Also, the processes for coating a bioabsorbable surgical article are not clearly described in the prior art. That is, the process of this invention uses a copolymer manufactured from at least about 50 percent by weight of the monomer caprolactone and the remainder glycolide. Copolymers of these proportions are soluble in acetone, as contrasted with, for example, at least some if not all of the copolymers of lactide and glycolide discussed in the prior art.

The use of a copolymer of caprolactone and glycolide as a suture is known in the prior art.

The use of a copolymer of at least 90% by weight caprolactone and another biodegradable monomer, e.g. glycolide, as a coating is disclosed in the prior art. The prior art also discloses a copolymer and a homopolymer of ε-caprolactone and as a suture coating, respectively.

The bioabsorbable coating of this invention has superior and unexpected results over the known commercially available surgical suture or ligature coatings. For example, the coating of this invention does not present a hazy appearance on a suture. The coating can be dissolved in acetone which seems to be less deleterious than other known solvents, for example, methylene chloride. Further, suture characteristics such as knot-snug-in or repositioning, knot security, and tissue drag appear to be equal to, if not better than suture coatings disclosed in the prior art.

The copolymers described in this invention are random. The term random copolymer means the result of a copolymerization reaction in which all of the monomers are charged into the reactor simultaneously. It is to be understood that variations in reaction conditions can lead to some differences in the actual degree of randomness with respect to the distribution of comonomer units in a copolymer chain.

According to the present invention, a surgical article having improved knot repositioning characteristics is provided, the article comprising a strand, the strand having a bioabsorbable coating, the coating selected from the group characterized by random copolymer from 65 up to 70% by weight of the copolymer consisting of linkages of formula (I):

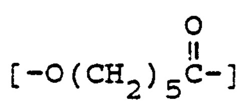

(I)

and the remaining linkages comprising at least one of the formulas (II) to (VIII):

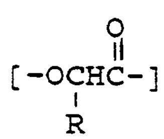

wherein R is H or $CH_3$ (II);

$$[-OCH_2CH_2CH_2O\overset{O}{\overset{\|}{C}}-] \qquad (III);$$

$$[-OCH_2CH_2OCH_2\overset{O}{\overset{\|}{C}}-] \qquad (IV);$$

$$[-O\underset{}{\overset{R'}{\overset{|}{C}}}HCH_2\overset{O}{\overset{\|}{C}}-] \quad \text{wherein R' is } CH_3 \text{ or } C_2H_5 \qquad (V);$$

$$[-O(CH_2)_4\overset{O}{\overset{\|}{C}}-] \qquad (VI);$$

$$[-O(CH_2)_XO\overset{O}{\overset{\|}{C}} - \overset{O}{\overset{\|}{C}} -] \quad \text{wherein X is 2 to 6} \qquad (VII);$$

$$[-O(CH_2)_XO\overset{O}{\overset{\|}{C}}CH_2\overset{O}{\overset{\|}{C}}-] \quad \text{wherein X is 2 to 6} \qquad (VIII);$$

or
characterized by a block copolymer having one or more A blocks solely manufactured from the monomer caprolactone, wherein the caprolactone in the one or more A blocks is 40 percent by weight of the copolymer, and one or more B blocks manufactured from: the monomer caprolactone randomly coplymerized with one or more monomers selected from the group consisting of lactides, carbonates and a lactone other than caprolactone, the total caprolactone linkages in the copolymer being from 70 to 80 percent by weight of said copolymer.

In one embodiment, the remaining linkages are selected from the group consisting of formulas (II) and (III). In another embodiment the remaining linkages are selected from the group consisting of formula (II).

In a specific embodiment, the formula (II) is:

$$[-O-CH_2 - \overset{O}{\overset{\|}{C}} -]$$

In another specific embodiment, the formula (II) is:

$$[-O-\underset{\underset{CH_3}{|}}{CH}-\overset{O}{\overset{\|}{C}}-]$$

In a further embodiment, the inherent viscosity of the copolymer is 0.2 to 1.4 dl/g (0.5 g/dl in $CHCl_3$, 30 °C) and the melting point is less than 50 °C.

It is to be understood that the term strand as used in this invention can be either a multifilament or a monofilament. A multifilament is preferred. The multifilament strand can be a braid.

The surgical article coated with the above described polymers can be bioabsorbable. In one embodiment, the bioabsorbable surgical article is a suture or ligature. In a specific embodiment, the suture or ligature is manufactured from a polymer prepared from one or more monomers selected from the group consisting of lactides, carbonates and lactones. If the polymer in a strand, for example a suture or ligature, and the copolymer in the coating are prepared from the same monomers, it is to be understood that the copolymer in the coating has a melting point of less than 50 °C or is noncrystalline, that is amorphous. It is to be further understood that this description applies by implication to the description of the invention in the claims.

In a more specific embodiment, the suture or ligature is manufactured from a homopolymer prepared from the monomer glycolide. In another more specific embodiment, the suture or ligature is manufactured from a homopolymer prepared from the monomer lactide. In yet another more specific embodiment, the suture or ligature is manufactured from a copolymer prepared from the monomers glycolide and 1,3-dioxan-2-one. In a further specific embodiment, the suture or ligature is manufactured from a copolymer prepared from the monomers glycolide and lactide.

The suture or ligature can be in multifilamentary form. In a specific embodiment, the coating comprises about 1/10 to 5% by weight of the coated multifilamentary suture or ligature. In a more specific embodiment, the coating comprises about 1/2 to 3% by weight of the coated multifilamentary suture or ligature. In the more specific embodiment, the coating can comprise up to about 1 1/2 percent by weight of the coated multifilamentary suture or ligature.

A process for coating a bioabsorbable surgical article has also been invented. The process comprises dissolving in acetone a random copolymer, from 65 to 70 percent by weight of the copolymer consists of linkages of formula (I):

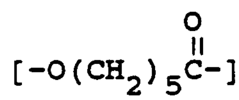

(I)

and the remaining linkages comprise at least one of the formulas (II) and (III):

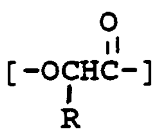

wherein R is H or $CH_3$ (II);

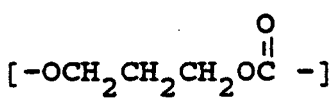

(III);

contacting the surgical article with the dissolved copolymer; maintaining the contact between the surgical article and dissolved copolymer until the copolymer on the surgical article comprises from about 1/10 to 5% by weight of the coated surgical article; removing the coated surgical article from the dissolved copolymer; and drying the copolymer coating on the surgical article. Parameters to be used in the maintaining step are fully described in the prior art. It is to be understood that the amount of pickup described in these patents can be increased or decreased by any person skilled in the art without undue experimentation. As a general statement, only one pass is required to obtain a coating comprising less than about 2 percent by weight of the coated strand. For a coating comprising more than about 2 percent to about 4 percent by weight of the coated strand, two passes are generally found to give a more uniform coating level. For coating levels from 4 to 5 percent, three passes will probably give the most uniform coating level.

In one embodiment, the caprolactone is $\epsilon$-caprolactone.

The term random or randomly in this invention means the result of a copolymerization reaction in which all of the monomers are charged into a reactor simultaneously. It is to be understood that variations in reaction conditions can lead to some differences in the actual degree of randomness with respect to the distribution of comonomer units in a copolymer chain.

A bioabsorbable coating for a surgical article comprising a block copolymer has also been invented. The block copolymer has one or more blocks manufactured from the monomer caprolactone. In one

embodiment, the caprolactone is ε-caprolactone. In another embodiment the copolymer is a diblock copolymer.

In a preferred embodiment, the copolymer has one or more A blocks solely manufactured from the monomer caprolactone, and one or more B blocks manufactured from one or more monomers selected from the group consisting of lactides, carbonates, lactones and oxalates, with the proviso that in the latter one or more B blocks, caprolactone can only be used with another monomer. In a further embodiment, the inherent viscosity of the block copolymer is about 0.1 to 1.0 dl/g (0.5 g/dl in $CHCl_3$, 30 ° C), the melting point of the block copolymer is less than 70 ° C, and the block copolymer is soluble in acetone or methylene chloride.

In a specific embodiment, the coating comprises a block copolymer consisting of one or more A blocks of formula (I):

$$[-O(CH_2)_5\overset{\overset{O}{\|}}{C}-] \qquad (I)$$

and the remaining one or more B blocks comprising one or more of the formulas (II) to (VI), either alone or in combination with formula (I):

$$[-O\underset{\underset{R}{|}}{C}H\overset{\overset{O}{\|}}{C}-] \quad \text{wherein R is H or } CH_3 \qquad (II);$$

$$[-OCH_2CH_2CH_2O\overset{\overset{O}{\|}}{C}-] \qquad (III);$$

$$[-OCH_2CH_2OCH_2\overset{\overset{O}{\|}}{C}-] \qquad (IV);$$

$$[-O\overset{\overset{R'}{|}}{C}HCH_2\overset{\overset{O}{\|}}{C}-] \quad \text{wherein R' is } CH_3 \text{ or } C_2H_5 \qquad (V);$$

$$[-O(CH_2)_4\overset{\overset{O}{\|}}{C}-] \qquad (VI),$$

the total amount of formula (I) in the copolymer being from 70 to 80 percent by weight of said copolymer.

In one embodiment, the remaining one or more B blocks consist of formulas (I) and (II). In another embodiment the remaining one or more B blocks consist of formulas (II) and (III).

In a specific embodiment, the formula (II) is:

$$[-O-CH_2-\overset{\overset{O}{\|}}{C}-].$$

The surgical article coated with the above-described polymers can be bioabsorbable. In one embodiment, the bioabsorbable surgical article is a suture or ligature. In a specific embodiment, the suture or ligature is manufactured from a polymer prepared from one or more monomers selected from the group

consisting of lactides, carbonates and lactones. If the polymer in the suture or ligature and the copolymer in the coating are prepared from the same monomers, it is to be understood that the copolymer in the coating has a melting point less than about 70 ° C and is soluble in acetone or methylene chloride. It is to be further understood that this description applies by implication to the description of the invention in the claims.

In a more specific embodiment, the suture or ligature is manufactured from a homopolymer prepared from the monomer glycolide. In another specific embodiment, the suture or ligature is manufactured from a polymer prepared from at least the monomer lactide. In yet another more specific embodiment, the suture or ligature is manufactured from a copolymer prepared from the monomers glycolide and 1,3-dioxan-2-one. In a further specific embodiment, the suture or ligature is manufactured from a copolymer prepared from the monomers glycolide and lactide.

The suture or ligature can be in multifilamentary form. In a specific embodiment, the coating comprises about 1/10 to 5% by weight of the coated multifilamentary suture or ligature. In a more specific embodiment, the coating comprises about 1/4 to 3% by weight of the coated multifilamentary suture or ligature. In the more specific embodiment, the coating can comprise up to about 2 1/2 percent by weight of the coated multifilamentary suture or ligature.

A coating process for a bioabsorbable surgical article comprises dissolving in acetone a block copolymer having one or more A blocks manufactured solely from the monomer caprolactone and one or more B blocks manufactured from the monomers caprolactone and glycolide, where the glycolide in the one or more B blocks comprises up to 50 percent by weight of the copolymer and up to about 65 percent by weight of said B blocks; contacting the surgical article with the dissolved copolymer; maintaining the contact between the surgical article and the dissolved copolymer until the copolymer on the article comprises from about 1/10 to 5% by weight of the coated surgical article; removing the coated surgical article from the dissolved copolymer; and drying the copolymer coating on the surgical article.

In one embodiment, the caprolactone is $\epsilon$-caprolactone.

## DESCRIPTION

### I. Random Copolymer Coating

The following examples describe the best mode of making and using the random copolymer coatings of this invention. Unless otherwise specified, all of the inherent viscosity ($\eta$inh) measurements in the examples were conducted at 30 ° C. Inherent viscosities are expressed in units of deciliters per gram (dl/g). The solution concentrations used to measure $\eta$inh are expressed in units of grams of polymer per deciliter of solution and are set in parentheses following the $\eta$inh value. The solvents used were either chloroform ($CHCl_3$) or hexafluoroacetone sesquihydrate (HFAS).

A description of inherent viscosity using the nomenclature described above is disclosed in the prior art.

### COMPARATIVE EXAMPLE 1

#### $\epsilon$-Caprolactone Homopolymer

A sample of $\epsilon$-caprolactone homopolymer was purchased from Scientific Polymer Products, Inc. The sample $\eta$inh was measured as 0.27 dl/g (0.5 g/dl in $CHCl_3$). GPC analysis in $CH_2Cl_2$ using polystyrene standards gave MW = 17,600 and MN = 8500.

### COMPARATIVE EXAMPLE 2

#### Synthesis of $\epsilon$-Caprolactone Homopolymer

$\epsilon$-Caprolactone (10g, 0.088 mole), lauryl alcohol (0.122g, 6.57 x $10^{-4}$ mole) and stannous chloride dihydrate (0.988 mg, 4.38 x $10^{-6}$ mole) were combined in a flask. The flask was flushed with nitrogen and evacuated. The flask was heated at 135 ° C in an oil bath for 24 hours. The resulting polymer had an $\eta$inh of 0.53 dl/g (0.5 g/dl in HFAS). GPC analysis in $CH_2Cl_2$ using polystyrene standards gave MW = 65,200 and MN = 26,900.

EXAMPLE 3

Synthesis of ε-Caprolactone-l-Lactide Copolymer

ε-Caprolactone (212.5g, 1.86 mole), l-lactide (37.5 g, 0.26 mole), lauryl alcohol (4.10 ml, 0.018 mole) and stannous chloride dihydrate (35.9 mg, 1.59 x $10^{-4}$ mole) were combined in a stirred reactor under nitrogen at 175 °C. The mixture was stirred at 175 °C for 3 hours. The resulting polymer had a composition, as determined by $^1H$ NMR, of 84 wt. % ε-caprolactone and 16 wt. % l-lactide. The inherent viscosity of the copolymer was 0.50 dl/g (0.5 g/dl in $CHCl_3$).

EXAMPLE 4

Synthesis of ε-Caprolactone-l-Lactide Copolymer

ε-Caprolactone (30.0 g, 0.26 mole), l-lactide (170.0g, 1.18 mole), lauryl alcohol (5.10g, 2.74 x $10^{-2}$ mole) and stannous chloride dihydrate (0.0162 g, 7.2 x $10^{-5}$ mole were combined in a stirred reactor under nitrogen at 180 °C. The mixture was stirred at 180 °C for 3 hours. The resulting polymer had a composition, as determined by $^1H$ NMR, of 13 wt. % ε-caprolactone and 87 wt. % l-lactide. The $\eta$inh was 0.27 dl/g (0.5 g/dl in $CHCl_3$).

EXAMPLE 5

Synthesis of ε-Caprolactone-Trimethylene Carbonate Copolymer

ε-Caprolactone (8.0 g, 0.070 mole), trimethylene carbonate (2.0 g, 0.020 mole), lauryl alcohol (0.283 g, 1.52 x $10^{-3}$ mole) and stannous chloride dihydrate (2.02 mg, 8.91 x $10^{-6}$ mole) were combined in a flask. The flask was flushed with nitrogen, evacuated and sealed. The flask was heated at 135 °C for 24 hours. The resulting polymer had a composition, as measured by $^1H$ NMR, of 86 wt. % ε-caprolactone and 14 wt. % trimethylene carbonate. The $\eta$inh of the copolymer was 0.26 dl/g (0.5 g/dl in HFAS).

EXAMPLE 6

Synthesis of ε-Caprolactone-Trimethylene Carbonate Copolymer

ε-Caprolactone (40 g, 0.35 mole), trimethylene carbonate (10 g. 0.098 mole), lauryl alcohol (1.42 g, 7.6 x $10^{-3}$ mole) and stannous chloride dihydrate (10.1 mg, 4.5 x $10^{-5}$ mole) were combined in a flask and heated for 24 hours under nitrogen at 135 °C. The resulting polymer had an inherent viscosity of 0.42 dl/g (0.5 g/dl in HFAS). The composition was determined by $^1H$ NMR to be 86 wt. % caprolactone and 14 wt. % trimethylene carbonate.

EXAMPLE 7

Synthesis of ε-Caprolactone-Glycolide Copolymer

ε-Caprolactone (170 g, 1.49 mole), glycolide (30 g, 0.26 mole), lauryl alcohol (1.37 g, 7.3 x $10^{-3}$ mole) and stannous octoate (0.052 g, 1.2 x $10^{-4}$ mole) were combined in a stirred reactor under nitrogen at 180 °C. The mixture was stirred at 180 °C for 4.5 hours. The resulting polymer had an inherent viscosity of 0.68 dl/g (0.5 g/dl in $CHCl_3$). The composition was determined by $^1H$ NMR to be 85 wt. % ε-caprolactone and 15 wt.% glycolide.

EXAMPLES 8-19

Synthesis of Caprolactone-Glycolide Copolymers

A series of random copolymers of ε-caprolactone and glycolide was prepared by the general procedure described in example 7. The bulk polymerizations were carried out in a stirred reactor under a slow nitrogen purge. The resulting polymers were dried in vacuum to remove residual monomer.

Specific preparative details and properties of the resulting polymers are summarized in Table 1.

**Table 1**

**Caprolactone/Glycolide Copolymers**

| Example | Composition as Charged: -Caprolactone grams | -Caprolactone moles | Glycolide grams | Glycolide moles | Stannous Octoate mg. | Stannous Octoate moles ($x10^{-3}$) | Lauryl Alcohol grams | Lauryl Alcohol moles ($x10^{-3}$) | Reaction Conditions Hrs | Reaction Conditions °C | Analyzed Properties: NMR[3] Cap (wt%) | NMR[3] Gly (wt%) | inh[4] $CHCl_3$ | DSC(5) Tm(°C) |
|---|---|---|---|---|---|---|---|---|---|---|---|---|---|---|
| 8 | 170 | 1.49 | 30 | 0.26 | 28.0[1] | 12.4 | 4.10 | 220 | 3 | 175 | 84.1 | 15.9 | 0.34 | 42 |
| 9 | 170 | 1.49 | 30 | 0.26 | 18.5 | 4.57 | 0.163 | 0.875 | 4.5 | 180 | 85.2 | 14.8 | 0.82 | 48 |
| 10 | 127.5 | 1.12 | 22.5 | 0.194 | 38.7 | 9.56 | 0.113 | 0.606 | 5.75 | 180 | 84.7 | 15.3 | 1.10 | 38 |
| 11 | 127.5 | 1.12 | 22.5 | 0.194 | 38.7 | 9.56 | 0.122 | 0.655 | 5.0 | 175 | 84.3 | 15.7 | 1.39 | NC[2] |
| 12 | 95.0 | 0.832 | 5.0 | 0.0431 | 26.0 | 6.41 | 0.652 | 3.50 | 3.0 | 175 | 94.8 | 5.2 | 0.77 | 55 |
| 13 | 90.0 | 0.789 | 10.0 | 0.0862 | 25.8 | 6.38 | 0.652 | 3.50 | 3.0 | 175 | 89.4 | 10.6 | 0.73 | 48 |
| 14 | 87.5 | 0.767 | 12.5 | 0.108 | 26.0 | 6.41 | 0.652 | 3.50 | 3.0 | 175 | 86.8 | 13.2 | 0.70 | 46 |
| 15 | 85.0 | 0.745 | 15.0 | 0.129 | 26.0 | 6.41 | 0.652 | 3.50 | 3.0 | 165 | 84.3 | 15.7 | 0.70 | 41 |
| 16 | 80.0 | 0.701 | 20.0 | 0.172 | 25.9 | 6.40 | 0.650 | 3.49 | 3.0 | 177 | 79.1 | 20.9 | 0.72 | NC |
| 17 | 70.0 | 0.613 | 30.0 | 0.258 | 25.9 | 6.40 | 0.649 | 3.48 | 3.0 | 176 | 68.6 | 31.4 | 0.60 | NC |
| 18 | 50.0 | 0.438 | 50.0 | 0.431 | 25.7 | 6.35 | 0.647 | 3.47 | 3.0 | 176 | 47.0 | 53.0 | 0.48 | NC |
| 19 | 40.0 | 0.350 | 60.0 | 0.517 | 25.7 | 6.35 | 0.646 | 3.47 | 3.0 | 175 | 36.3 | 63.7 | 0.73 (HFAS) | NC |

(1) $SnCl_2 \cdot 2H_2O$ was used as a catalyst in this sample.
(2) NC = Not crystalline at room temperature.
(3) NMR is Nuclear Magnetic Resonance Spectrometry.
(4) inh is inherent viscosity of a 0.5 g/dl solution of the polymer in the solvent. The solvents used were chloroform ($CHCl_3$) and hexafluoroacetone sesquihydrate (HFAS).
(5) DSC is Differential Scanning Calorimetry. Tm is the melting temperature (peak).

Table 2 summarizes the in vitro performance for the coatings of this invention.

## Table 2

## In Vitro Coating Performance

| Suture Coated with Example | Wt.% Cap in Copolymer | Wt.% Coating(1) | Knot Run-down(2) Wet | Snug-in Work(3) (kg mm) | Security Work(4) (kg mm) |
|---|---|---|---|---|---|
| Control | 0 | 0 | L | - | - |
| 1 | 100 | 1 | R/L | - | - |
| | | 2 | RC | - | - |
| | | 3 | RC | - | - |
| 2 | 100 | 1 | L | - | - |
| | | 2 | L | - | - |
| | | 3 | L | - | - |
| 3 | 84 | 0.2 | R | - | - |
| | | 0.3 | R | - | - |
| | | 0.5 | R | - | - |
| | | 0.6 | R | - | - |
| | | 1.0 | R | - | - |
| | | 1.2 | RW | - | - |
| | | 1.6 | RW | - | - |
| | | 2.4 | RW | - | - |
| 4 | 13 | 3 | L | - | - |
| 5 | 86 | 1 | RW | - | - |
| | | 2 | RW | - | - |
| | | 3 | RW | - | - |
| 6 | 86 | 1 | RW | - | - |
| | | 2 | RW | - | - |
| | | 3 | RW | - | - |
| 7 | 85 | 0.7 | RC | - | - |
| | | 1.1 | RW | - | - |
| | | 1.2 | RW | - | - |
| | | 1.7 | RW | - | - |
| 8 | 84.1 | 0.8 | RW | - | - |
| | | 1.5 | RW | - | - |
| | | 1.8 | RW | - | - |

Table 2 (continued)

In Vitro Coating Performance

| Suture Coated with Example | Wt.% Cap in Copolymer | Wt.% Coating(1) | Knot Run-down(2) Wet | Snug-in Work(3) (kg mm) | Security Work(4) (kg mm) |
|---|---|---|---|---|---|
| 9 | 85.2 | 0.2 | - | - | - |
| | | 0.9 | RC | - | - |
| | | 1.6 | RW | - | - |
| | | 2.2 | RW | - | - |
| 10 | 84.7 | 0.8 | - | - | - |
| | | 1.5 | - | - | - |
| | | 2.1 | - | - | - |
| 11 | 84.3 | 1.3 | RU | - | - |
| | | 2.1 | RD/L | - | - |
| | | 3.0 | RD | - | - |
| 12 | 94.8 | 0.72 | RC | 29.16 ± 3.18 | 22.08 ± 3.14 |
| | | 1.53 | RC | 26.24 ± 1.55 | 22.02 ± 0.54 |
| | | 2.11 | RC | 21.77 ± 2.78 | 23.82 ± 1.04 |
| 13 | 89.4 | 0.75 | R | 12.24 ± 8.53 | 20.85 ± 1.43 |
| | | 1.54 | R | 13.55 ± 11.00 | 22.28 ± 0.98 |
| | | 2.28 | RW | 20.33 ± 7.90 | 21.49 ± 1.11 |
| 14 | 86.8 | 0.76 | R | 4.66 ± 0.87 | 15.39 ± 1.76 |
| | | 1.49 | RW | 7.12 ± 1.65 | 17.89 ± 0.75 |
| | | 2.24 | RW | 8.46 ± 3.34 | 19.25 ± 0.75 |
| 15 | 84.3 | 0.76 | RW | 7.56 ± 3.75 | 12.46 ± 2.05 |
| | | 1.49 | RW | 4.37 ± 1.95 | 14.79 ± 1.74 |
| | | 2.22 | RW | 9.94 ± 4.10 | 14.50 ± 1.96 |
| 16 | 79.1 | 0.81 | RW | 1.82 ± 0.98 | 9.79 ± 1.75 |
| | | 1.59 | RW | 1.99 ± 0.59 | 11.16 ± 1.44 |
| | | 2.36 | RW | 2.03 ± 0.98 | 11.90 ± 1.90 |
| 17 | 68.6 | 0.73 | RW | 4.73 ± 3.21 | 13.83 ± 1.26 |
| | | 1.36 | R | 1.52 ± 0.49 | 14.12 ± 1.47 |
| | | 2.07 | R | 5.35 ± 3.62 | 14.61 ± 1.20 |
| 18 | 47.0 | 0.71 | RD/RC | 29.39 ± 1.81 | 20.09 ± 1.99 |
| | | 1.38 | RD/RC | 31.15 ± 0.88 | 20.61 ± 1.50 |
| | | 2.06 | RD/RC | 28.02 ± 1.58 | 18.84 ± 1.72 |
| 19 | 36.3 | 1.71 | RD | 44.39 ± 1.30 | 23.09 ± 0.45 |
| | | 3.07 | L/RU | 45.47 ± 2.90 | 23.67 ± 0.33 |
| | | 4.54 | L/RU | 39.20 ± 5.21 | 23.59 ± 0.49 |

TABLE 2 FOOTNOTES

(1) The coatings were applied by hand dipping 1/0 polyglycolic acid braid in a 3% (wt./vol.) polymer solution in acetone for examples 1, 2, 4, 5, and 6. Coating levels for these examples were rounded to the nearest integer. Examples 3, 7, 8, 9, 10, and 11 were coated from a 2% (wt./vol.) solution in acetone using a capillary coating machine. The remainder of the samples were machine coated from a 3.5% (wt./vol.) solution in acetone or methylene chloride (example 19 only).

(2) Square knots were formed in the coated 1/0 polyglycolic acid braid using a conventional suture tying board. The knot was then run down to the board to assess the stick-slipping of the knot (chatter) as it runs down and to assess the force required to initiate and sustain the run-down. The abbreviations are: R, Runs; L, Locks; RC, Runs with Chatter; RD, Runs with Difficulty; RU, Runs with Unpredictability; RW, Runs Well. The comparisons are made on suture wet with saline.

(3) This test measures the ability of a suture (in this case 1/0 polyglycolic acid) to be snugged-in. A loop is passed around a steel rod and tied with a square knot. The knot is set to a prescribed tension with an Instron tester, and the tension is then removed. After resetting the gage length and removing the steel rod, the loop is tested to break. The force and crosshead movement are recorded by an attached computer which calculates the work needed to move the crosshead 15 mm. Samples were tested immediately after 30 seconds

immersion in saline solution (0.9% NaCl in distilled water).

The tensions used to set the knots, and all the other conditions of knot tying and testing, are practical laboratory conditions, but may not correspond to actual surgical practice. The knot snug-in may not correlate with clinical experience.

(4) A strand (in this case 1/0 polyglycolic acid) is tied to itself to form a loop using a square + 1 knot. The second and third throws of the knot are set to a prescribed tension, the loop is cut, and the cut ends are clamped in the jaws of an Instron tester. In the same way as was described above for snug-in, the work required to move the crosshead 10 mm is determined. Samples are tested immediately after 30 seconds immersion in saline solution.

Table 3 summarizes the in vivo performance for some of the bioabsorbable coatings of this invention.

TABLE 3

IN VIVO COATING EVALUATIONS[1]

| Coating Polymer From: | Wt.% Coating[2] | Knot Repositioning Ability[3] | Knot Security[4] Category: 1 | 2 |
|---|---|---|---|---|
| Control | 0 | 0/8 | 4/4 | 0/4 |
| Example 3 | 0.6 | 7/18 | 14/14 | 0/14 |
| | 1.2 | 12/18 | 13/17 | 4/17 |
| | 1.8 | 10/18 | 16/18 | 2/18 |
| | 2.4 | 8/18 | 15/17 | 2/17 |
| Example 7 | 1.2 | 31/33 | 17/32 | 15/32 |
| | 1.8 | 27/27 | 20/27 | 7/27 |
| Example 9 | 1.7 | 24/24 | 15/24 | 9/24 |

## TABLE 3 FOOTNOTES

(1) Coated, needled and sterilized sutures were tested in dogs.

(2) The coatings were applied to 1/0 polyglycolic acid braid from a 2% (wt./vol.) solution of the coating material dissolved in acetone.

(3) A suture coated with the test material is passed through two sides of a wound in the animal. A square knot is formed in the suture approximately 12-15 mm from the final knot position required to close the wound. The two ends of the suture are then pulled to slide the knot into position. Knots that slide properly are rated 1 while knots that fail to move into position are rated 0. The rating for a coating is the sum of the "1" ratings divided by the total number of test specimens.

(4) Immediate knot security is determined by using a pair of curved tweezers to tug at the 8 to 10 mm length of the ears of a square knot with two additional throws. Knots that are secure when manipulated are rated 1, knots with a loose top throw are rated 2, knots with an open top throw are rated 3, and knots that are not secure when manipulated are rated 4. The number of knots falling into each category is then divided by the total number of test specimens to provide a rating in each category. No values for categories 3 or 4 were reported for these examples.

II. Block Copolymer Coating

The following examples describe the best mode of making and using the block copolymer coatings of this invention. Unless otherwise specified, all of the inherent viscosity ($\eta$inh) measurements in the examples were conducted at 30°C. Inherent viscosities are expressed in units of deciliters per gram (dl/g). The solution concentrations used to measure $\eta$inh are expressed in units of grams of polymer per deciliter of solution and are set in parentheses following the $\eta$inh value. The solvents used were either chloroform

($CHCl_3$) or hexafluoroacetone sesquihydrate (HFAS).

A description of inherent viscosity using the nomenclature described above is disclosed in the prior art.

It is to be understood that an increase in the inherent viscosity of the copolymer will provide for an increase in the knot security but also an increase in the absorption time of the coated surgical article and in the work required to reposition a knot.

The term block copolymer described in this invention means a copolymer with a non-random distribution of comonomer units along the chain. A convenient shorthand notation to describe different block architectures utilizes alphabetic symbols for the individual block segments. The number and types of comonomer units within a block segment can be specified. For example, AB represents a diblock copolymer, ABA or BAB represents a triblock copolymer. More complex architectures such as tetrablocks or pentablocks, etc., can also be described using this notation, e.g. ABABA. A multiblock copolymer can be represented as $\{AB\}_n$. If more than two types of blocks are present, additional alphabetic symbols can be defined.

Block copolymers are formed in this invention by the sequential addition of comonomers or mixtures of comonomers to a reactor. It is believed that with this type of preparation method, there can be a distribution of block size and block composition and that the chain architecture can be compromised by ester interchange reactions.

## EXAMPLE 20

### Synthesis of ε-Caprolactone-(ε-Caprolactone-Glycolide) AB Block Copolymer

ε-Caprolactone (80g, 0.70 mole), lauryl alcohol (1.585 ml, 6.6 x $10^{-3}$ mole) and stannous octoate (39.5 ul, 1.22 x $10^{-4}$ mole) were combined in a stirred reactor at 180 °C. The mixture was stirred while the temperature was increased to 200 °C over 8 min. and stirring was continued for 1.5 hours. Glycolide (60g, 0.52 mole) and ε-Caprolactone (60g, 0.53 mole) were added and the temperature was reduced to 180 °C. Stirring was maintained for 2 hours longer. The copolymer was discharged from the reactor and was dried in a vacuum oven for 24 hours at about 50 °C to remove residual monomer. The analyzed properties of the resulting copolymer are listed in Table 4, Section A.

## EXAMPLES 21-33

### Synthesis of ε-Caprolactone-(ε-Caprolactone-Glycolide) AB Block Copolymers

A series of ε-caprolactone-glycolide copolymers was prepared by the general procedure described in Example 20, although the polymerization temperature was maintained at about 180 °C throughout the reaction in these examples. Specific preparative details and properties of the resulting copolymers are summarized in Table 4, Section A.

## EXAMPLES 34-37

### Synthesis of ε-Caprolactone-(Glycolide-1,3-Dioxan-2-one) AB Block Copolymers

A series of copolymers containing ε-caprolactone, glycolide and 1,3-dioxan-2-one were prepared by the same general procedure described in Example 20, with the exception that the second charge contained glycolide and 1,3-dioxan-2-one. The temperature was held at about 180 °C throughout the reaction in each of these examples. Specific preparative details and properties of the resulting copolymers are summarized in Table 4, Section B.

**Table 4, Section A**

**Caprolactone/Glycolide Copolymer Properties**

| Example | Polymerization Conditions: 1st Charge Cap[1] | 1st Charge Time(hrs) | 2nd Charge Cap[1] | 2nd Charge Gly[1] | 2nd Charge Time(hrs) | Mole % LA[2] | Mole % T-9[3] | NMR[4] Wt % Cap | NMR[4] $\bar{L}_c$ | NMR[4] $\bar{L}_g$ | DSC[5] Tm °C | DSC[5] Δ H Cal/g | $\eta_{inh}$[6] $CHCl_3$ | Solubility in Acetone |
|---|---|---|---|---|---|---|---|---|---|---|---|---|---|---|
| 20 | 40 | 1.5 | 30 | 30 | 2.0 | 0.40 | .007 | 69.6 | 2.98 | 2.78 | 58 | 10.1 | 0.54 | Gels > 4% |
| 21 | 40 | 1.5 | 30 | 30 | 3.0 | 1.53 | .01 | 70.1 | 2.62 | 2.20 | 56 | 8.5 | 0.27 | Sol. |
| 22 | 40 | 1.5 | 30 | 30 | 3.0 | 2.29 | .01 | 70.6 | 2.46 | 2.22 | 54 | 7.9 | 0.22 | Sol. |
| 23 | 40 | 1.5 | 30 | 30 | 3.0 | 4.65 | .01 | 73.2 | 2.57 | 2.08 | 47 | 8.2 | 0.13 | Sol. |
| 24 | 30 | 1.5 | 42 | 28 | 2.5 | 1.15 | .01 | 72.0 | 2.22 | 1.75 | 55 | 6.2 | 0.34 | Sol. |
| 25 | 30 | 1.5 | 42 | 28 | 3.0 | 1.72 | .01 | 71.9 | 2.14 | 1.73 | 42 | 5.4 | 0.27 | Sol. |
| 26 | 20 | 1.5 | 40 | 40 | 2.0 | 0.40 | .01 | 57.9 | 1.85 | 2.75 | 54 | 6.7 | 0.51 | Sol/Hazy |
| 27 | 20 | 1.5 | 48 | 32 | 3.0 | 0.40 | .01 | 64.4 | 1.94 | 2.23 | 58 | 5.0 | 0.46 | Sol. |
| 28 | 20 | 1.5 | 48 | 32 | 3.0 | 1.15 | .007 | 68.0 | 1.80 | 1.77 | 50 | 3.7 | 0.33 | Sol. |
| 29 | 20 | 1.5 | 48 | 32 | 3.0 | 0.77 | .01 | 67.0 | 1.82 | 1.84 | 55 | 4.1 | 0.39 | Sol. |
| 30 | 20 | 1.5 | 52 | 28 | 2.0 | 0.40 | .01 | 71.6 | 1.99 | 1.57 | 56 | 5.4 | 0.59 | Sol. |
| 31 | 10.5 | 1.5 | 58.2 | 31.3 | 3.0 | 0.40 | .007 | 66.0 | 1.66 | 1.71 | 56 | 2.4 | 0.55 | Sol. |
| 32 | 10.5 | 1.5 | 53.7 | 35.8 | 3.0 | 0.40 | .007 | 61.3 | 1.55 | 2.04 | 55 | 2.4 | 0.33 | Sol. |
| 33 | 3.5 | 1.5 | 62.7 | 33.8 | 2.5 | 0.40 | .007 | 63.3 | 1.50 | 1.71 | 42 | 0.5 | 0.55 | Sol. |

(1) Numbers under these categories represent the Wt % of the added monomer with respect to the weight of the total copolymer.
(2) Lauryl Alcohol as initiator.
(3) Stannous Octoate catalyst purchased from M&T Chemicals.
(4) Nuclear Magnetic Resonance Spectrometry. $\bar{L}_c$ is the average segment length of consecutive caprolactone units in the polymer. $\bar{L}_g$ is the average segment length of glycolide units.
(5) Differential scanning calorimetry. Tm is the melting temperature (peak), Δ H is the heat of fusion.
(6) Inherent viscosity of a 0.5 g/dl solution of polymer in chloroform.

**Table 4, Section B**

**Caprolactone/Glycolide/1,3-Dioxan-2-one Copolymer Properties**

| Example | Charged Composition | | | | | | | NMR(4) | | | DSC(5) | | ηinh(6) | Solubility in $CH_2Cl_2$ |
|---|---|---|---|---|---|---|---|---|---|---|---|---|---|---|
| | 1st Charge | | 2nd Charge | | | Mole % | | Wt % | | | | | | |
| | Cap(1) | Time(hrs) | GLY(1) | TMC(1) | Time(hrs) | LA(2) | T-9(3) | Cap | Gly | TMC | Tm °C | ΔH Cal/g | $CHCl_3$ | |
| 34 | 20 | 1.5 | 40 | 40 | 2.0 | 0.40 | .01 | 18.9 | 41.6 | 39.5 | 51 | 2.8 | 0.39 | Sol. |
| 35 | 20 | 1.5 | 40 | 40 | 3.0 | 0.73 | .01 | 18.8 | 41.6 | 39.6 | 48 | 3.1 | 0.29 | Sol. |
| 36 | 20 | 1.5 | 40 | 40 | 3.0 | 1.10 | .01 | 17.5 | 41.5 | 41.0 | 49 | 3.0 | 0.26 | Sol. |
| 37 | 40 | 1.5 | 30 | 30 | 3.0 | 1.48 | .01 | 37.4 | 30.8 | 31.8 | 57 | 8.2 | 0.23 | Sol. |

(1) Numbers under these categories represent the Wt % of the added monomer with respect to the weight of the total copolymer. TMC is an abbreviation for trimethylene carbonate which is the same as 1,3 dioxan-2-one.
(2) Lauryl Alcohol as initiator.
(3) Stannous Octoate catalyst purchased from M&T Chemicals.
(4) Nuclear Magnetic Resonance Spectrometry.
(5) Differential scanning calorimetry. Tm is melting temperature (peak), ΔH is the heat of fusion.
(6) Inherent viscosity of a 0.5 g/dl solution of polymer in chloroform.

Table 5 summarizes the in vitro performance for the bioabsorbable coatings of this invention.

### Table 5, Section A

### In Vitro Coating Performance of Cap/Gly AB Copolymers

| Example | Wt. % (1) Coating | —15mm SNUG-IN— (2) WORK (Kg mm) | S. DEV. | —10mm SECURITY— (3) WORK (Kg mm) | S. DEV. | Hand Tested (4) Wet Knot Run Down |
|---|---|---|---|---|---|---|
| 20 | 0.75 | 3.31 | 1.93 | 18.96 | 2.28 | R |
| | 1.38 | 8.04 | 2.28 | 21.49 | 1.24 | R |
| | 1.91 | 17.80 | 4.61 | 21.68 | 1.72 | RW |
| 21 | 0.76 | 1.86 | 1.37 | 12.94 | 4.03 | RW |
| | 1.39 | 1.07 | 0.33 | 15.49 | 1.19 | R |
| | 2.24 | 3.02 | 0.75 | 17.52 | 1.77 | RW |
| 22 | 0.88 | 1.52 | 0.86 | 15.09 | 3.03 | R |
| | 1.37 | 4.00 | 3.16 | 14.94 | 2.12 | R |
| | 2.04 | 7.82 | 2.37 | 15.30 | 3.60 | RW |
| 23 | 0.55 | 1.58 | 0.50 | 15.32 | 2.55 | R |
| | 1.77 | 8.56 | 6.71 | 15.98 | 2.75 | R |
| | 1.91 | 5.99 | 2.91 | 17.63 | 0.94 | R |
| 24 | 0.80 | 3.37 | 4.96 | 11.61 | 4.23 | RW |
| | 1.44 | 3.12 | 2.21 | 12.29 | 2.89 | RW |
| | 2.00 | 1.87 | 0.60 | 12.25 | 3.81 | RW |
| 25 | 0.89 | 0.76 | 0.23 | 11.41 | 4.85 | R |
| | 1.34 | 1.08 | 0.19 | 12.61 | 3.83 | RW |
| | 1.93 | 2.26 | 2.23 | 15.08 | 2.02 | RW |
| 26 | 0.66 | 13.99 | 5.76 | 19.47 | 1.97 | RC |
| | 1.31 | 20.97 | 2.16 | 20.60 | 2.05 | RC/RD |
| | 1.95 | 22.53 | 4.78 | 21.07 | 1.77 | RC/RD |
| 27 | 0.70 | 9.17 | 3.70 | 15.24 | 1.83 | RC |
| | 1.25 | 7.56 | 2.53 | 14.57 | 2.19 | R |
| | 1.78 | 15.79 | 7.04 | 15.57 | 2.75 | RW |
| 28 | 0.75 | 0.85 | 0.29 | 11.78 | 1.99 | RW |
| | 1.38 | 1.46 | 0.31 | 11.61 | 2.95 | RW |
| | 1.83 | 3.10 | 3.12 | 11.78 | 2.79 | RW |
| 29 | 0.66 | 2.07 | 1.19 | 10.62 | 3.40 | R |
| | 1.38 | 3.96 | 1.35 | 13.29 | 1.95 | R |
| | 2.10 | 5.98 | 3.61 | 10.98 | 3.33 | RW |
| 30 | 0.72 | 1.91 | 0.62 | 13.64 | 3.76 | RW |
| | 1.41 | 8.03 | 2.82 | 13.59 | 3.50 | RW |
| | 2.06 | 10.22 | 2.07 | 13.69 | 3.35 | RW |

Table 5, Section A
(continued)

In Vitro Coating Performance of
Cap/Gly AB Copolymers

| Example | Wt. % Coating (1) | —15mm SNUG-IN— (2) WORK (Kg mm) | S. DEV. | —10mm SECURITY— (3) WORK (Kg mm) | S. DEV. | Hand Tested (4) Wet Knot Run Down |
|---|---|---|---|---|---|---|
| 31 | 0.61 | 7.79 | 5.53 | 14.06 | 3.21 | R |
| | 1.35 | 7.44 | 4.84 | 14.95 | 3.81 | R |
| | 1.94 | 9.17 | 1.47 | 16.02 | 1.88 | RW |
| 32 | 0.75 | 10.31 | 8.07 | 16.18 | 1.74 | R |
| | 1.38 | 16.66 | 3.76 | 16.49 | 1.56 | RW |
| | 1.95 | 14.39 | 1.28 | 17.44 | 2.01 | R |
| 33 | 0.78 | 13.36 | 3.04 | 16.58 | 2.90 | RC |
| | 1.50 | 16.46 | 11.46 | 14.81 | 2.46 | R |
| | 2.24 | 12.45 | 3.10 | 16.67 | 1.40 | RW |
| Uncoated Braid | — | 38.32 | 5.29 | 17.95 | 4.44 | L |

## Table 5 , Section B

### In Vitro Coating Performance of Cap/Gly/1.3-Dioxan-2-one AB Copolymers

| Example | # Dips(1) | Hand Tested Wet Knot Run Down(4) |
|---|---|---|
| 34 | 1 | R |
| | 2 | R |
| | 3 | R |
| 35 | 1 | R |
| | 2 | R |
| | 3 | R |
| 36 | 1 | R |
| | 2 | R |
| | 3 | R |
| 37 | 1 | R |
| | 2 | R |
| | 3 | R |

## TABLE 5 FOOTNOTES

(1) The coatings were applied to 1/0 polyglycolic acid braid from a 3.5% (wt/vol.) solution of the coating material dissolved in acetone using a capillary coating machine (all Section A copolymers) or hand dipped in a 3.0% (wt/vol) solution in methylene chloride (all Section B copolymers).

(2) This test measures the ability of a suture to be snugged-in. A loop is passed around a steel rod and tied with a square knot. The knot is set to a prescribed tension with an Instron tester, and the tension is then removed. After resetting the gage length and removing the steel rod, the loop is tested to break. The force and crosshead movement are recorded by an attached computer which calculates the work needed to move the crosshead 15 mm. Samples are tested immediately after 30 seconds immersion in saline solution (0.9% NaCl in distilled water). The number of specimens used in this test was 5, except for the uncoated braid, which used 3 specimens.
The tensions used to set the knots, and all the other conditions of knot tying and testing, are practical laboratory conditions, but may not correspond to actual surgical practice. The knot snug-in may not correlate with clinical experience.

(3) A strand is tied to itself to form a loop using a square + 1 knot. The second and third throws of the knot are set to a prescribed tension, the loop

is cut, and the cut ends are clamped in the jaws of an Instron tester. In the same way as was described above for snug-in, the work required to move the crosshead 10 mm is determined. Samples are tested immediately after 30 seconds immersion in saline solution. The number of specimens used in the test was 10.

(4) Square knots were formed in hand-dipped (Section B) or machine coated (Section A) 1/0 polyglycolic acid braid using a conventional suture tying board. The knot was then run down to the board to assess the stick-slipping of the knot (chatter) as it runs down and to assess the force required to initiate and sustain the run-down. The abbreviations are: R, Runs; L, Lock; RC, Runs with Chatter; RD, Runs with Difficulty; RU, Runs with Unpredictability; RW, Runs Well. The comparisons are made on suture wet with saline.

Table 6 summarizes the in vivo performance for some of the bioabsorbable coatings of this invention.

## Table 6

### In Vivo Coating Evaluations[1]

| Coating Polymer From: | Wt. %[2] Coating | Knot Repositioning[3] Ability | Knot Security[4] Category: A | B |
|---|---|---|---|---|
| Uncoated Braid | 0 | 0/8 | 4/4 | 0/4 |
| Example 20 | 0.94 | 4/5 | 5/5 | 0/5 |
| | 1.54 | 4/5 | 5/5 | 0/5 |
| | 2.57 | 5/5 | 5/5 | 0/5 |
| Example 21 | 1.39 | 10/10 | 9/10 | 1/10 |
| | 2.24 | 10/10 | 9/10 | 1/10 |
| Example 22 | 1.37 | 10/10 | 10/10 | 0/10 |
| | 2.04 | 10/10 | 7/10 | 3/10 |
| Example 23 | 1.77 | 10/10 | 10/10 | 0/10 |
| | 1.91 | 10/10 | 10/10 | 0/10 |
| Example 24 | 1.34 | 10/10 | 10/10 | 0/10 |
| | 1.93 | 10/10 | 9/10 | 1/10 |

TABLE 6 FOOTNOTES

(1) Coated, needled and sterilized sutures were tested in dogs.

(2) The coatings were applied to 1/0 polyglycolic acid braid from a 3.5% (wt/vol) solution of the coating material dissolved in acetone using a capillary coating machine.

(3) A suture coated with the test material is passed through two sides of a wound in the animal. A square knot is formed in the suture approximately 12-15 mm from the final knot position required to close the wound. The two ends of the suture are then pulled to slide the knot into position. Knots that slide properly are rated 1 while knots that fail to move into position are rated 0. The rating for a coating is the sum of the "1" ratings divided by the total number of test specimens.

(4) Immediate knot security is determined by using a pair of curved tweezers to tug at the 8 to 10 mm length of the ears of a square knot with two additional throws. Knots that are secure when manipulated are rated 1, knots with a loose top throw are rated 2, knots with an open top throw are rated 3, and knots that are not secure when manipulated are rated 4. The number of knots falling into each category is then divided by the total number of test specimens to provide a rating in each category. Samples rated 1 or 2 are designated A in the table, those rated 3 or 4 are designated B.

EXAMPLE 38

Synthesis of ε-Caprolactone-1-Lactide AB Block Copolymer

ε-Caprolactone (55 g, 0.482 mole), lauryl alcohol (0.148 g, 7.92 x $10^{-4}$ mole) and stannous chloride dihydrate (7.19 mg, 3.19 x $10^{-5}$ mole) were combined in a stirred reactor at 154°C. The mixture was stirred for 2 hours at 162°C to 172°C. 1-Lactide (83 g, 0.58 mole) was added and the temperature was gradually increased to 220°C. The mixture was stirred for 1 hour. More 1-lactide (77 g, 0.53 mole) was added. The mixture was stirred for 1 hour. The resulting copolymer ηinh was 1.15 dl/g (0.5 g/dl in HFAS). The composition, as measured by $^1$H NMR was 27 wt. % caprolactone and 73 wt. % lactide.

EXAMPLE 39

Synthesis of ε-Caprolactone-1-Lactide AB Block Copolymer

ε-Caprolactone (112 g, 0.98 mole), lauryl alcohol (0.193 g, 8.5 x $10^{-4}$ mole) and stannous chloride dihydrate (19.15 mg, 8.5 x $10^{-5}$ mole) were conbined in a stirred reactor at 162°C. The mixture was stirred at 162°C. for 6 hours. The temperature was increased to 180°C. and 16 g of 1-lactide was added (0.11 mole). The temperature was gradually increased to 220°C. over 1 hour and then 84 g 1-lactide was added. The mixture was stirred for 45 min. The resulting polymer had an inherent viscosity of 1.26 dl/g (0.5 g/dl in HFAS). The composition was determined by $^1$H NMR to be 53 wt. % caprolactone and 47 wt. % lactide.

EXAMPLE 40

Synthesis of ε-Caprolactone-1-Lactide ABA Block Copolymer

ε-Caprolactone (55 g, 0.482 mole), diethylene glycol (0.201 g, 1.90 x $10^{-3}$ mole) and stannous chloride dihydrate (7.19 mg, 3.19 x $10^{-5}$ mole) were combined in a stirred reactor at 154°C. The mixture was stirred for 2 hours at 162°C. to 172°C. 1-Lactide (20 g, 0.14 mole) and stannous chloride dihydrate (7.19 mg, 3.19 x $10^{-5}$ mole) were added and the temperature was gradually increased to 220°C. The mixture was stirred for 0.5 hours. More 1-lactide (140 g, 0.97 mole) was added. The mixture was stirred for 1 hour. The resulting copolymer ηinh was 1.29 dl/g (0.5 g/dl in HFAS). The composition, as measured by $^1$H NMR, was 26 wt. % caprolactone and 74 wt. % lactide.

Table 7 summarizes the in vitro performance for the bioabsorbable coatings of this invention.

TABLE 7

IN VITRO COATING PERFORMANCE

| Coating Polymer From: | #Dips(1) | Hand Tested Wet Knot Run Down(2) |
|---|---|---|
| Example 38 | 1 | L |
| Example 39 | 1 | RD |
| Example 40 | 1 | L |

## TABLE 7 FOOTNOTES

(1) The coatings were applied to 1/0 polyglycolic acid braid by hand dipping in a 2% (wt/vol.) solution of the coating material dissolved in methylene chloride.

(2) Square knots were formed in hand-dipped 1/0 polyglycolic acid braid using a conventional suture tying board. The knot was then run down to the board to assess the stick-slipping of the knot (chatter) as it runs down and to assess the force required to initiate and sustain the run-down. The abbreviations are: R, Runs; L, Locks; RC, Runs with Chatter; RD, Runs with Difficulty; RU, Runs with Unpredictability; RW, Runs Well. The comparisons are made on suture wet with saline.

## Claims

**1.** A surgical article having improved knot repositioning characteristics, the article comprising a strand, the strand having a bioabsorbable coating, the coating selected from the group characterized by a random copolymer, from 65 up to 70 percent by weight of the copolymer consisting of linkages of formula (I):

$$[-O(CH_2)_5\overset{\overset{O}{\|}}{C}-] \qquad (I)$$

and the remaining linkages comprising at least one of the formulas (II) to (VIII):

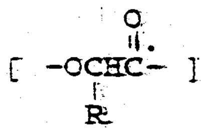 wherein R is H or $CH_3$ (II);

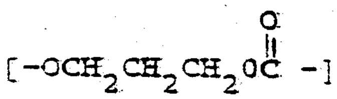 (III);

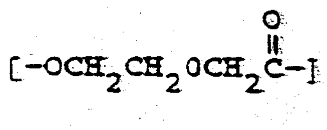 (IV);

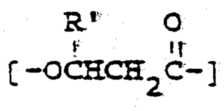 wherein R' is $CH_3$ or $C_2H_5$ (V);

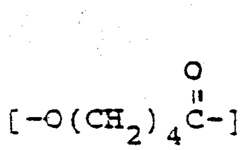 (VI);

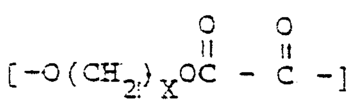 wherein X is 2 to 6 (VII); and

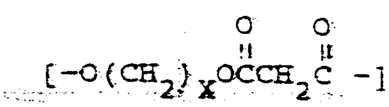 wherein X is 2 to 6 (VIII); or

characterized by a block copolymer having one or more A blocks solely manufactured from the monomer caprolactone, wherein the caprolactone in the one or more A blocks is 40 percent by weight of the copolymer, and one or more B blocks manufactured from the monomer caprolactone randomly copolymerized with one or more monomers selected from the group consisting of lactides, carbonates and a lactone other than caprolactone, the total caprolactone linkages in the copolymer being from 70 to 80 percent by weight of said copolymer.

2. The article of claim 1 characterized by a random copolymer, wherein said remaining linkages are selected from the group consisting of formulas (II) and (III).

3. The article of claim 2 comprising a surgical suture or ligature having a bioabsorbable, multifilamentary strand manufactured from a polymer prepared from one or more monomers selected from the group consisting of glycolide, lactide, 1,3-dioxan-2-one and 1,4-dioxan-2-one, the coating characterized by a random copolymer, wherein the remaining linkages comprise at least the formula (II):

$$[-OCH_2\overset{O}{\overset{\|}{C}}-] \qquad (II).$$

**4.** The article of claim 2 or 3 wherein the inherent viscosity of said coating copolymer is 0.2 to 1.4 dl/g (0.5 g/dl in $CHCl_3$, 30 ° C).

**5.** The article of claim 1 comprising a surgical suture or ligature having a bioabsorbable, multifilamentary strand manufactured from a polymer prepared from one or more monomers selected from the group consisting of glycolide, lactide, 1,3-dioxan-2-one and 1,4-dioxan-2-one, the coating characterized by a block copolymer, the total caprolactone linkages in the block copolymer being 70 percent by weight of said copolymer, wherein the inherent viscosity of the coating copolymer is 0.4 to 0.8 dl/g (0.5 g/gl in $CHCl_3$, 30 ° C).

**6.** The article of claim 1 or 2 or 3 or 5 wherein said coating comprises 1/10 to 5 percent by weight of the coated strand.

**7.** A process for coating the article of claim 3 comprising dissolving in acetone the random copolymer;
contacting the suture or ligature with the dissolved copolymer,
maintaining the contact between said suture or ligature, and said dissolved copolymer until the copolymer comprises from 1/10 to 5% by weight of the coated suture or ligature;
removing said coated suture or ligature from said dissolved copolymer; and
drying the coating on said suture or ligature.

**8.** A process for coating the article of claim 1 comprising a bioabsorbable, multifilamentary surgical suture or ligature comprising:
dissolving in acetone the block copolymer having one or more A blocks solely manufactured from the monomer caprolactone and one or more B blocks manufactured from the monomers caprolactone and glycolide, wherein the glycolide in the one or more B blocks comprises up to 50 percent by weight of the copolymer and up to 65 percent by weight of said B blocks;
contacting the suture or ligature with the dissolved copolymer;
maintaining the contact between said suture or ligature, and said dissolved copolymer until the copolymer on said suture or ligature comprises from 1/10 to 5% by weight of the coated suture or ligature;
removing said coated suture or ligature from said dissolved copolymer; and
drying the copolymer coating on said suture or ligature.

## Patentansprüche

**1.** Chirurgischer Gegenstand mit einer verbesserten Eigenschaft, Knoten zu reponieren, wobei der Gegenstand einen Faden umfaßt, der Faden eine bioabsorbierbare Beschichtung aufweist, die Beschichtung ausgewählt ist aus der Gruppe, die durch ein statistisches Copolymer gekennzeichnet ist, wobei 65 bis 70 Gewichtsprozent des Copolymers aus Verbindungen der Formel (I):

$$[-O(CH_2)_5\overset{O}{\overset{\|}{C}}-] \qquad (I)$$

bestehen und die verbleibenden Verbindungen mindestens eine der Formeln (II) bis (VIII) umfassen:

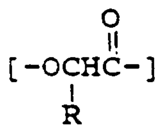 worin R H oder $CH_3$ ist (II);

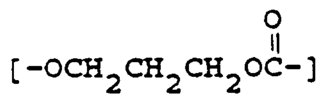 (III);

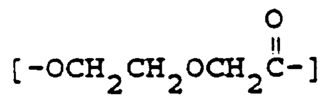 (IV);

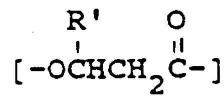 worin R′ $CH_3$ oder $C_2H_5$ ist (V);

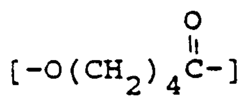 (VI),

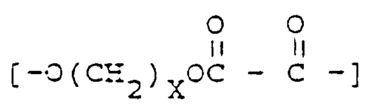 worin X 2 bis 6 ist (VII); und

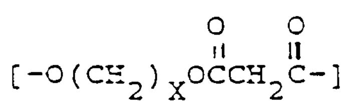 worin X 2 bis 6 ist (VIII); oder

gekennzeichnet sind durch ein Block-Copolymer mit einem oder mehreren Blöcken A, die allein aus dem Monomer Caprolacton hergestellt sind, worin das Caprolacton in dem einen oder mehreren Blöcken A 40 Gewichtsprozent des Copolymers ausmacht, und einem oder mehreren Blöcken B, die aus dem Monomer Caprolacton hergestellt sind, welches statistisch mit einem oder mehreren Monomeren copolymerisiert ist, die aus der aus Lactiden, Carbonaten und einem von Caprolacton verschiedenen Lacton bestehenden Gruppe ausgewählt sind, wobei die Gesamt-Caprolacton-Verbindungen in dem Copolymer von 70 bis 80 Gewichtsprozent des Copolymers betragen.

2. Gegenstand nach Anspruch 1, gekennzeichnet durch ein statistisches Copolymer, worin die verbleibenden Verbindungen aus der aus den Formeln (II) und (III) bestehenden Gruppe ausgewählt sind.

3. Gegenstand nach Anspruch 2, umfassend ein chirurgisches Nahtmaterial oder Abbindungsmaterial mit einem bioabsorbierbaren vielfaserigen Faden, der aus einem Polymer hergestellt ist, welches aus einem oder mehreren Monomeren hergestellt ist, die aus der aus Glycolid, Lactid, 1,3-Dioxan-2-on und 1,4-Dioxan-2-on bestehenden Gruppe ausgewählt sind, wobei die Beschichtung gekennzeichnet ist durch ein statistisches Copolymer, worin die verbleibenden Verbindungen mindestens die Formel (II) umfassen:

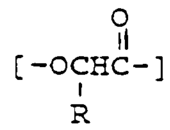

(II).

4. Gegenstand nach Anspruch 2 oder 3, worin die Grenzviskosität des Beschichtungscopolymers 0,2 bis 1,4 dl/g (0,5 g/dl in $CHCl_3$, 30 ° C) beträgt.

5. Gegenstand nach Anspruch 1, umfassend ein chirurgisches Nahtmaterial oder Abbindungsmaterial mit einem bioabsorbierbaren vielfaserigen Faden, hergestellt aus einem Polymer, das aus einem oder mehreren Monomeren hergestellt ist, die aus der aus Glycolid, Lactid, 1,3-Dioxan-2-on und 1,4-Dioxan-2-on bestehenden Gruppe ausgewählt sind, wobei die Beschichtung durch ein Block-Copolymer gekennzeichnet ist, wobei die gesamten Caprolacton-Verbindungen in dem Block-Copolymer 70 Gewichtsprozent des Copolymers ausmachen, worin die Grenzviskosität des Beschichtungscopolymers 0,4 bis 0,8 dl/g (0,5 g/gl in $CHCl_3$, 30 ° C) beträgt.

6. Gegenstand nach Anspruch 1 oder 2 oder 3 oder 5, worin die Beschichtung 1/10 bis 5 Gewichtsprozent des beschichteten Fadens umfaßt.

7. Verfahren zum Beschichten des Gegenstands nach Anspruch 3, umfassend das Auflösen des statistischen Copolymers in Aceton;
das Inkontaktbringen des Nahtmaterials oder Abbindungsmaterials mit dem gelösten Copolymer;
das Aufrechterhalten des Kontakts zwischen dem Nahtmaterial oder Abbindungsmaterial und dem gelösten Copolymer, bis das Copolymer 1/10 bis 5 Gew.-% des beschichteten Nahtmaterials oder Abbindungsmaterials umfaßt;
das Entfernen des beschichteten Nahtmaterials oder Abbindungsmaterials aus dem gelösten Copolymer; und
das Trocknen der Beschichtung auf dem Nahtmaterial oder Abbindungsmaterial.

8. Verfahren zum Beschichten des Gegenstands nach Anspruch 1, umfassend ein bioabsorbierbares, vielfaseriges chirurgisches Nahtmaterial oder Abbindungsmaterial, umfassend:
das Auflösen in Aceton des Block-Copolymers mit einem oder mehreren Blöcken A, die allein aus dem Monomer Caprolacton hergestellt sind, und einem oder mehreren Blöcken B, die aus den Monomeren Caprolacton und Glycolid hergestellt sind, worin das Glycolid in dem einen oder mehreren Blöcken B bis zu 50 Gewichtsprozent des Copolymers und bis zu 65 Gewichtsprozent der Blöcke B umfaßt;
das Inkontaktbringen des Nahtmaterials oder Abbindungsmaterials mit dem gelösten Copolymer;
das Aufrechterhalten des Kontakts zwischen dem Nahtmaterial oder Abbindungsmaterial und dem gelösten Copolymer, bis das Copolymer auf dem Nahtmaterial oder Abbindungsmaterial 1/10 bis 5 Gew.-% des beschichteten Nahtmaterials oder Abbindungsmaterials umfaßt;
das Entfernen des beschichteten Nahtmaterials oder Abbindungsmaterials aus dem gelösten Copolymer; und
das Trocknen der Copolymer-Beschichtung auf dem Nahtmaterial oder Abbindungsmaterial.

**Revendications**

1. Article chirurgical possédant des caractéristiques améliorées de repositionnement du noeud, l'article comprenant un fil, le fil possédant un enrobage bioabsorbable, l'enrobage étant choisi dans le groupe caractérisé par un copolymère statistique, de 65 à 70% en poids du copolymère étant constitué de motifs de formule (I) :

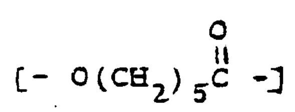

(I)

et les motifs restant comprenant au moins les formules (II) et (VIII) :

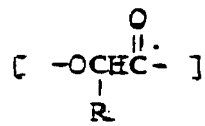 dans laquelle R est H ou $CH_3$ (II);

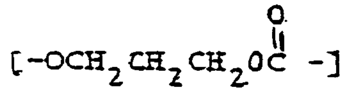 (III) ;

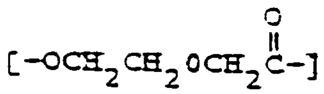 (IV) ;

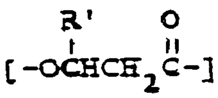 dans laquelle R' est $CH_3$ ou $C_2H_5$ (V);

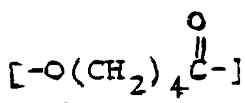 (VI) :

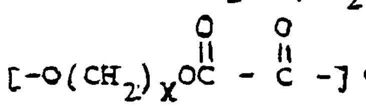 dans laquelle X est de 2 à 6 (VII);

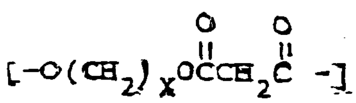 dans laquelle X est de 2 à 6 (VIII); ou

caractérisé par un copolymère séquencé possédant un ou plusieurs blocs A fabriqués seulement à partir du monomère caprolactone, dans lequel la caprolactone dans un ou plusieurs des blocs A représente 40% en poids du copolymère, et un ou plusieurs blocs B fabriqués à partir du monomère caprolactone copolymérisé statistiquement avec un ou plusieurs monomères choisis dans le groupe constitué par les lactides, les carbonates et une lactone autre que la caprolactone, le total des motifs caprolactone dans le copolymère représentant de 70 à 80% en poids dudit copolymère.

2. Article selon la revendication 1, caractérisé par un copolymère statistique, dans lequel lesdits motifs restants sont choisis dans le groupe constitué par les formules (II) et (III).

3. Article selon la revendication 2, comprenant une suture ou une ligature chirurgicale possédant un fil bioabsorbable à plusieurs filaments fabriqué à partir d'un polymère préparé à partir d'un ou plusieurs monomères choisis dans le groupe constitué par le glycolide, le lactide, la 1,3-dioxane-2-one et la 1,4-dioxane-2-one, l'enrobage étant caractérisé par un copolymère statistique, dans lequel les motifs restants comprennent au moins la formule (II) :

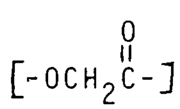 (II).

4. Article selon la revendication 2 ou 3, dans lequel la viscosité inhérente dudit copolymère d'enrobage est de 0,2 à 1,4 dl/g (0,5 g/dl dans $CHCl_3$, 30 ° C).

5. Article selon la revendication 1, comprenant une suture ou une ligature chirurgicale possédant un fil bioabsorbable à plusieurs filaments fabriqué à partir d'un polymère préparé à partir d'un ou plusieurs monomères choisis dans le groupe constitué par le glycolide, le lactide, la 1,3-dioxane-2-one et la 1,4-dioxane-2-one, l'enrobage étant caractérisé par un copolymère séquencé, le total des motifs caprolactone dans le copolymère séquencé étant de 70% en poids dudit copolymère, dans lequel la viscosité inhérente dudit copolymère d'enrobage est de 0,4 à 0,8 dl/g (0,5 g/dl dans $CHCl_3$, 30 °C).

**6.** Article selon la revendication 1 ou 2 ou 3 ou 5, dans lequel ledit enrobage constitue 0,1 à 5% en poids du fil enrobé.

**7.** Procédé pour enrober l'article de la revendication 3, comprenant la dissolution dans de l'acétone du copolymère statistique;

la mise en contact de la suture ou de la ligature avec le copolymère dissous;

le maintien du contact entre ladite suture ou ladite ligature et ledit copolymère dissous jusqu'à ce que le copolymère constitue de 0,1 à 5% en poids de la suture ou de la ligature enrobée;

l'enlèvement de ladite suture ou de ladite ligature enrobée dudit copolymère dissous; et

le séchage de l'enrobage sur ladite suture ou ladite ligature.

**8.** Procédé pour enrober l'article de la revendication 1 comprenant une suture ou une ligature chirurgicale bioabsorbable à plusieurs filaments, comprenant :

la dissolution dans l'acétone du copolymère séquencé constitué d'un ou plusieurs blocs A fabriqués seulement à partir du monomère caprolactone, et d'un ou plusieurs blocs B fabriqués à partir des monomères caprolactone et glycolide, où le glycolide contenu dans les un ou plusieurs blocs B constitue jusqu'à 50% en poids du copolymère et jusqu'à 65% en poids desdits blocs B;

la mise en contact de la suture ou de la ligature avec le copolymère dissous;

le maintien du contact entre ladite suture ou ladite ligature et ledit copolymère dissous jusqu'à ce que le copolymère sur ladite suture ou ladite ligature constitue de 0,1 à 5% en poids de la suture ou de la ligature enrobée;

l'enlèvement de ladite suture ou de ladite ligature enrobée du copolymère dissous; et

le séchage de l'enrobage de copolymère sur ladite suture ou ladite ligature.